Europäisches Patentamt

European Patent Office

Office européen des brevets

(11) Publication number: **0 602 619 A1**

## EUROPEAN PATENT APPLICATION

(21) Application number: 93120199.0

(51) Int. Cl.5: **A61K 9/28**, A61K 31/44

(22) Date of filing: **15.12.93**

(30) Priority: **18.12.92 IT MI922885**

(43) Date of publication of application:
**22.06.94 Bulletin 94/25**

(84) Designated Contracting States:
**AT BE CH DE DK ES FR GB GR IE IT LI LU MC NL PT SE**

(71) Applicant: **IdB Holding S.p.A.**
**Via Ripamonti, 99**
**I-20141 Milano(IT)**

(72) Inventor: **Seghizzi, Roberto**
**Via Ripamonti 99**
**20141 Milano(IT)**
Inventor: **Vitali, Romano**
**Via Ripamonti 99**
**20141 Milano(IT)**
Inventor: **Zini, Lugi**
**Via Ripamonti 99**
**20141 Milano(IT)**
Inventor: **Pifferi, Giorgio**
**Via Ripamonti 99**
**20141 Milano(IT)**

(74) Representative: **Minoja, Fabrizio**
**Studio Consulenza Brevettuale,**
**Via Rossini, 8**
**I-20122 Milano (IT)**

(54) **Controlled-release pharmaceutical compositions containing nicergoline.**

(57) Oral controlled-release pharmaceutical compositions are described, containing as active principle nicergoline or a pharmaceutically acceptable salt thereof, comprising mixtures of microgranules loaded with nicergoline or salt thereof and coated with a film consisting of:
- 100-97% of a neutral copolymer having average molecular weight of 800,000 consisting of ethyl acrylate and methyl methacrylate;
- 0-3% of hydrosoluble substances.

EP 0 602 619 A1

The present invention refers to oral controlled-release pharmaceutical formulations containing nicergoline as the active principle. Nicergoline or 8-$\beta$-1,6-dimethyl-[(5-bromonicotinoyloxy)methyl]-10$\alpha$-methoxyergoline, is a semi-synthetic ergoline derivative.

Nicergoline and its active metabolites favourably influence the cerebral hemodynamic and metabolism. These activities are due to the $\alpha$-lytic and myolytic activities and to the increase in glucose and oxygen consumption. The drug has also platelet anti-aggregation activity.

Nicergoline is absorbed in the intestinal tract, rapidly metabolized by hydrolysis, followed by partial demethylation and/or conjugation with glucuronic acid. It is mainly excreted by the kidneys in a very similar way for each animal species considered: rat, dog, monkey, man.

In humans, the administered dose is excreted in metabolized form, mainly in the urine and in a minor amount (10%) in the feces in a period of 3-4 days. Nicergoline is used for the clinical treatment of acute and chronic cerebro-vascular metabolic disorders due to arteriosclerosis thrombosis, cerebral embolism and transient cerebral ischemia.

The conventional oral formulations require two or more daily administrations. A once-a-day slow release oral formulation would bring about remarkable advantages in terms of convenience for the patient and of the compliance improvement. Moreover, it is known that from the therapeutical point of view a controlled-release formulation allows to maintain more constant blood concentrations, thus decreasing possible side-effects.

It has now been found, according to the present invention, that an effective slow-release of nicergoline or of its pharmaceutically acceptable salts can be obtained by means of pharmaceutical compositions comprising mixtures of microgranules loaded with nicergoline or its salts and coated with a film consisting of:

- 100-97% of a neutral copolymer having average molecular weight of 800,000 consisting of ethyl acrylate and methyl methacrylate;
- 0-3% of hydrosoluble substances.

The multiparticulate controlled-release formulations of the invention allow the oral administration of a single daily-dose of nicergoline in amounts ranging from 20 to 100 mg, preferably from 40 to 80 mg.

Nicergoline is loaded on microgranules preferably as a salt of a suitable pharmaceutically acceptable organic acid, such as tartaric or succinic acid. Nicergoline and the organic acid are present in ratios from 1:0.5 to 1:3 (mole:mole), preferably in stoichiometric ratio.

The loading medium is preferably aqueous and may also contain binding, wetting, anti-adherent and anti-foam agents. The loading suspension is applied on inert microgranules consisting of starch and sucrose, having sizes ranging from 0.4 to 1.4 mm, preferably from 0.6 to 0.9 mm.

The release of nicergoline from the loaded microgranules is controlled by the coating with the acrylic copolymer film commercially available under the trademark Eudragit Ne.

Films made of this polymer are insoluble in water and in the gastrointestinal fluids, permeable to aqueous media and are swellable. These peculiar characteristics make this polymer particulary suited to the preparation of nicergoline "reservoir" microgranules able to control the release by diffusion through the coating membrane. The nicergoline release through the polymeric film decreases as its thickness increases and it is therefore possible to decrease the release rate by applying layers of increasing thickness.

The polymeric coating is applied to the microgranules loaded with nicergoline in amounts ranging from 4 to 20%, preferably from 6 to 16% (w/w). The aqueous suspension of the coating polymer may also contain anti-adherent and anti-foaming agents.

Moreover, the release rate of the active principle can be decreased by using inert microgranules of increasing sizes from 0.4 to 1.4 mm.

According to a further object of the invention, the coating film controlling the nicergoline release may consist of a mixture of an ethyl acrylate and methyl methacrylate copolymer with suitable water-soluble substances to obtain a microporous coating film. In such a system, the active principle is mainly released through the porosities formed in the film by the dissolution of the soluble substance when it comes into contact with the gastro-intestinal fluids. The film porosity is determined by the hydrosoluble substance/copolymer ratio: the higher this ratio, the faster the nicergoline release for equal film thickness.

Examples of water soluble substances comprise polyvinylpyrrolidones, polyethylene glycols, hydroxypropyl methylcellulose, lactose, sucrose or mixtures thereof, preferably hydroxypropyl methylcellulose in percentages to the polymer ranging from 1 to 3% (w/w).

When the film consists of water-soluble substances and copolymers, it is applied on the nicergoline microgranules in amounts ranging from 10 to 30% (w/w). The coating suspension may be added with plasticizing, anti-adherent and anti-foaming agents.

2

A third object of the invention is that the nicergoline release can be further programmed by mixing microgranules having different release rates of the active principle. This allows a remarkable flexibility in the multiparticulate formulation so that the most suited release pattern may be obtained.

For instance, non coated, fast-release microgranules loaded with nicergoline amounts from 5 to 25 mg, can be mixed with controlled-release microgranules obtained according to the first two objects of the invention, loaded with nicergoline amounts ranging from 15 to 75 mg.

The loading of microgranules with nicergoline, the coating with the polymeric film and the drying may be carried out using fluid-bed or rotary pan apparatuses.

The microgranules may then be filled into capsules or formulated as tablets.

The following examples further illustrate the invention.

Example 1

Gelatine capsules containing 60 mg nicergoline.
Each capsule contains:

| | |
|---|---|
| Nicergoline | 60 mg |
| Sucrose | 130.1 mg |
| Talc | 58.4 mg |
| Maize starch | 21.2 mg |
| Ethyl acrylate/methyl methacrylate copolymer | 26 mg |
| Tartaric acid | 18.6 mg |
| Colloidal silicon dioxide | 8.5 mg |
| Polyvinylpyrrolidone | 3.75 mg |
| Polysorbate 80 | 1.25 mg |
| Simethicone emulsion | 0.20 mg. |

600 g of nicergoline and 186 g of tartaric acid are dissolved in 1700 g of purified water at 70°C.

37.5 g of polyvinylpyrrolidone and 12.5 g of polysorbate 80 are added to this solution, stirring until complete dissolution. 6.25 g of 16% simethicone emulsion, 225 g of talc and 25 g of colloidal silicon dioxide are then added, homogeneously dispersing with a turbine type stirrer.

The obtained suspension is sprayed on 1513 g of inert starch and sucrose microgranules of sizes ranging from 0.6 to 0.85 mm in fluid-bed apparatus, then the microgranules are dried overnight in a static oven at 40°C.

For the coating of the microgranules, 339 g of talc, 6.25 g of 16% simethicone emulsion and 40 g of colloidal silicon dioxide are dispersed in 1900 g of purified water with a turbine type stirrer. The suspension is deaerated and 867 g of a 30% ethyl acrylate/methyl methacrylate copolymer suspension are added under continuous stirring.

The obtained suspension is sprayed on the microgranules previously loaded with nicergoline, in a fluid bed apparatus.

The coated microgranules are dried in a static oven at 40°C overnight.

The coated microgranules are mixed with 20 g of colloidal silicon dioxide and 20 g of talc, then filled into capsules, dosing at 328 mg/capsule.

Example 2

Gelatine capsules containing 60 mg of nicergoline.
Each capsule contains:

| Nicergoline | 60 mg |
| Sucrose | 115.1 mg |
| Talc | 58.4 mg |
| Maize starch | 36.2 mg |
| Ethyl acrylate/methyl methacrylate copolymer | 26 mg |
| Tartaric acid | 18.6 mg |
| Colloidal silicon dioxide | 8.5 mg |
| Polyvinylpyrrolidone | 3.75 mg |
| Polysorbate 80 | 1.25 mg |
| Simethicone emulsion | 0.20 mg. |

The preparation is carried out as in Example 1, using inert microgranules of a larger size than those described in Example 1, ranging from 0.95 to 1.2 mm.

The microgranules are filled into capsules, dosing at 328 mg/capsule.

Example 3

Gelatine capsules containing 60 mg of nicergoline.

Each capsule contains:

| Nicergoline | 60 mg |
| Sucrose | 115.1 mg |
| Talc | 38.46 mg |
| Maize starch | 36.2 mg |
| Tartaric acid | 18.6 mg |
| Ethyl acrylate/methyl methacrylate copolymer | 10.4 mg |
| Colloidal silicon dioxide | 6.1 mg |
| Polyvinylpyrrolidone | 3.75 mg |
| Polysorbate 80 | 1.25 mg |
| Simethicone emulsion | 0.14 mg. |

The preparation is carried out as in Example 2, using a thinner polymeric film. The microgranules are filled into capsules, dosing at 290 mg mg/capsule.

Example 4

Gelatine capsules containing 60 mg of nicergoline.

Each capsule contains:

| Nicergoline | 60 mg |
| Sucrose | 130.1 mg |
| Talc | 90.32 mg |
| Ethyl acrylate/methyl methacrylate copolymer | 52 mg |
| Maize starch | 21.2 mg |
| Tartaric acid | 18.6 mg |
| Colloidal silicon dioxide | 12.2 mg |
| Polyvinylpyrrolidone | 3.75 mg |
| Polysorbate 80 | 1.25 mg |
| Hydroxypropyl methylcellulose | 1.07 mg |
| Simethicone emulsion | 0.3 mg |
| Polyethylene glycol 6000 | 0.21 mg. |

The loading of nicergoline is carried out as in Example 1.

For the coating of microgranules with a microporous polymeric film, 2.1 g of polyethylene glycol 6000 and 10,7 g of hydroxypropyl methylcellulose are dissolved in 3760 g of purified water at 90°C under vigorous stirring. 12.5 g of 16% simethicone emulsion, 82 g of colloidal silicon dioxide and 663 g of talc are added to this solution, homogeneously dispersing with a turbine type stirrer. The mixture is cooled and 1733 g of a 30% ethyl acrylate and methyl methacrylate copolymer suspension are added under continuous stirring.

The obtained solution is sprayed on microgranules previously loaded with nicergoline in a fluid bed apparatus.

The loaded microgranules are mixed with 15 g colloidal silicon dioxide and 15 g of talc, then filled into capsules, dosing at 391 mg/capsule.

## Example 5

Gelatine capsules containing 60 mg of nicergoline.
Each capsule contains:

| | |
|---|---|
| Nicergoline | 60 mg |
| Sucrose | 130.1 mg |
| Talc | 49.42 mg |
| Maize starch | 21.2 mg |
| Ethyl acrylate/methyl methacrylate copolymer | 19.5 mg |
| Tartaric acid | 18.6 mg |
| Colloidal silicon dioxide | 7 mg |
| Polyvinylpyrrolidone | 3.75 mg |
| Polysorbate 80 | 1.25 mg |
| Simethicone emulsion | 0.18 mg. |

The loading of nicergoline is carried out as in Example 1.

For the coating with microgranules, 254.2 g of talc, 5 g of 16% simethicone emulsion and 30 g of colloidal silicon dioxide are dispersed in 1420 g of purified water, with a turbine type stirrer. The suspension is deaerated and 650 g of a 30% ethyl acrylate/methyl methacrylate copolymer suspension are added under continuous stirring. The obtained suspension is sprayed on 1950 g of microgranules previously loaded with nicergoline in a fluid bed apparatus. The coated microgranules are dried overnight in a static oven at 40°C. Non coated microgranules (650 g, corresponding to 150 g of nicergoline) are mixed with 2430 g of coated microgranules (corresponding to 450 g of nicergoline), 15 g of colloidal silicon dioxide and 15 g of talc. The microgranule mixture is filled into capsules, dosing at 311 mg/capsule.

## Example 6

Gelatine capsules containing 60 mg of nicergoline (reference preparation).
Each capsule contains:

| | |
|---|---|
| Nicergoline | 60 mg |
| Sucrose | 128 mg |
| Talc | 36.44 mg |
| Maize starch | 20.9 mg |
| Tartaric acid | 18.6 mg |
| Ammonium methacrylate copolymer, type B | 18 mg |
| Colloidal silicon dioxide | 4 mg |
| Triethyl citrate | 4 mg |
| Polyvinylpyrrolidone | 3.75 mg |
| Ammonium methacrylate copolymer, type A | 2 mg |
| Polysorbate 80 | 1.25 mg |
| Simethicone emulsion | 0.06 mg. |

37.5 g of polyvinylpyrrolidone and 12.5 g of polysorbate 80 are added to a solution of 600 g of nicergoline and 186 g of tartaric acid in 1490 g of purified water at 70°C and 150 g of talc and 25 g of colloidal silicon dioxide are dispersed therein. The obtained suspension is sprayed on 1490 g of inert starch and sucrose microgranules (of sizes ranging from 0.6 to 0.85 mm) in a fluid-bed apparatus, with final drying in an oven at 40°C overnight.

Separately, 40 g of triethyl citrate are dissolved in 1300 g of purified water at 70°C, subsequently dispersing 4 g of a simethicone emulsion (16%) and 199.4 g of talc. The mixture is cooled and 66.8 g of a 30% suspension of ammonium methacrylate copolymer type A and 600 g of a 30% suspension of ammonium methacrylate type B, are added under continuous stirring. The obtained suspension is sprayed on the microgranules previously loaded with nicergoline, in a fluid bed apparatus. The coated microgranules are dried in a static oven at 40°C overnight, then mixed with 15 g colloidal silicon dioxide and 15 g of talc and filled into capsules dosing at 297 mg/capsule.

This capsule formulation containing microgranules coated with a mixture of cation copolymers instead of neutral copolymers, has been used as reference in the dissolution tests.

The release characteristics of the formulations of Examples 1-6 have been evaluated by the dissolution test with an apparatus according to Ph. Eur. II, provided with paddle stirrer. Artificial gastric juice (pH 1.2) was used as dissolution medium for the first two hours, then pH was raised to pH 6.5 with phosphate buffer.

The results of the dissolution tests, expressed in Table 1 as released nicergoline percent in comparison to the nominal content of the capsules, show that:

- the release of the active principle may be protracted using a neutral acrylic copolymer as the coating film (Examples 1-4), but not with cationic acrylic copolymers (Example 6);
- the release may be modulated either by applying polymeric films of different thickness (Examples 2 and 3) or using inert microgranules of different sizes (Examples 1 and 2)
- the release may also be made faster by making the coating film microporous through incorporation of suitable hydrosoluble substances (Example 4 vs. Example 1);
- finally, the release may be suitably programmed and made sustained by mixing, for example, different microgranules having different release rates (example 5 vs. 1).

## Table 1

% Nicergoline released from capsules

| Time (hours) | Example 1 | Example 2 | Example 3 | Example 4 | Example 5 | Example 6 |
|---|---|---|---|---|---|---|
| 0.5 | 0.1 | 0 | 1.2 | 0.3 | 25.9 | 4.6 |
| 2 | 21.7 | 9.6 | 30.1 | 32.0 | 37.7 | 70.8 |
| 4 | 49.7 | 26.3 | 54.0 | 64.0 | 58.3 | 78.5 |
| 6 | 66.7 | 43.2 | 67.6 | 75.0 | 71.7 | 80.0 |
| 8 | 74.1 | 56.0 | 73.4 | 81.1 | 78.2 | 81.9 |
| 16 | 85.1 | 72.3 | 83.9 | 87.4 | 86.4 | 84.4 |

The results of the dissolution test on the multiparticulate formulations of Examples 1-5, confirm the possibility of prolonging and/or sustaining the nicergoline release with suitable coatings of a neutral acrylic copolymer.

A pharmacokinetic evaluation was carried out by administering "cross-over" the formulation of Example 5 to three healthy volunteers 26-35 year old in comparison to conventional 30 mg nicergoline tablets on the market. One conventional formulation tablet was first given to each fasting subject twice a day, at 9 a.m. and at 4 p.m., then, after "wash-out" for one week, one capsule of Example 5 was given at 9 a.m. Blood samples were drawn at time zero (before administration) and at 0,5, 1, 2, 3, 5, 8, 9, 10, 11, 13, 24 hours and placed in test-tubes containing sodium heparin. The samples were immediately centrifuged at 4°C, 3500 rpm for 10 minutes and the obtained plasma was frozen with acetone/ice and preserved at -20°C until the time of analysis. Since the nicergoline half-life is very short, its main metabolite, 10α-methoxydihydrolysergol (M2), was measured by a HPLC specific method using a reverse phase column, after purification by extraction on Extrelut cartridges and subsequent solvent extraction.

Table 2 – Plasma levels of M2 metabolite after oral administration of nicergoline in healthy volunteers

| Metabolite M2 (ng/ml) Time (hours) | 30 mg Tablet (2/die) | 60 mg Capsule (1/die) |
|---|---|---|
| 0 | 0 | 0 |
| 0.5 | 4.94 | 3.00 |
| 1 | 35.75 | 4.72 |
| 2 | 25.79 | 14.85 |
| 3 | 27.05 | 21.31 |
| 5 | 16.92 | 24.43 |
| 8 | 10.03 | 13.48 |
| 9 | 16.65 | 12.96 |
| 10 | 33.17 | 21.11 |
| 11 | 38.56 | 19.84 |
| 13 | 21.28 | 17.54 |
| 24 | 13.83 | 14.82 |

The results obtained (Table 2) show that the formulation of Example 5, administered once a day, provides up to 24 hours a plasma level of the M2 active metabolite comparable to those of the conventional formulation administered twice a day. In addition to the better patient compliance due to the single daily administration, the formulations of the invention allow to reduce the peak concentrations and consequently the entity of nicergoline side-effects, such as hypotension and vertigo, gastric disturbances, hot flushes and reddening of the skin.

**Claims**

1. Oral controlled-release pharmaceutical compositions containing as active principle nicergoline or a pharmaceutically acceptable salt thereof, comprising mixtures of microgranules loaded with nicergoline or a salt thereof and coated with a film consisting of:
   - 100-97% of a neutral copolymer having average molecular weight of 800,000 consisting of ethyl acrylate and methyl methacrylate;
   - 0-3% of hydrosoluble substances.

2. Compositions according to claim 1, in which the inert microgranules loaded with nicergoline or a salt thereof are starch and sucrose microgranules with sizes from 0.4 to 1.4 mm.

3. Compositions according to claim 2, in which the microgranules have a size ranging from 0.6 to 0.9 mm.

4. Compositions according to any one of claims 1 to 3, comprising microgranules different in size and coating, so as to provide controlled release rates of the active principle.

5. Compositions according to any one of claims 1 to 4, in which the film consists of a 100% neutral ethyl acrylate/methyl methacrylate copolymer having molecular weight of 800,000.

6. Compositions according to claim 5, in which the film is in amounts from 4 to 20 w/w of the composition.

7. Compositions according to any one of claims 1 to 4, in which the film consists of a 97-99% neutral ethyl acrylate/methyl methacrylate copolymer and of a 1-3% hydrosoluble substance.

8. Compositions according to claim 7 in which the hydrosoluble substance is selected from polyvinylpyrrolidones, polyethylene glycols, hydroxypropyl methylcellulose, lactose, sucrose or mixtures thereof.

9. Compositions according to claim 7 or 8, wherein the film is from 10 to 30% w/w of the composition.

10. Compositions comprising suitable mixtures of coated microgranules according to claims 1 to 9 with non coated microgranules loaded with nicergoline or a salt thereof.

11. Compositions according to any one of previous claims containing from 20 to 100 mg of nicergoline.

12. Capsules or tablets comprising microgranules according to any one of claims 1 to 11.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | BR. J. CLIN. PHARMACOL., 1993, 35/1 (80P) UNITED KINGDOM Johnston A. et al 'Bioequivalence of controlled release nicergoline 60 mg once daily vs the plain form 30 mg twice daily in 24 male subjects' * the whole document * | 1-12 | A61K9/28 A61K31/44 |
| Y | SCI. TECH. PRAT. PHARM., 1990, 6/SPEC. ISS. (134-137) FRANCE Liddiard C. 'Monograph in preparation: Polymethacrylates' * page 135, right column; table II * | 1-12 | |
| Y | US-A-5 015 480 (RAY F. CHILDERS ET AL.) * column 1 - column 5 * | 1-12 | |

TECHNICAL FIELDS SEARCHED (Int.Cl.5)

A61K

The present search report has been drawn up for all claims

| Place of search | Date of completion of the search | Examiner |
|---|---|---|
| MUNICH | 14 March 1994 | Tzschoppe, D |

EPO FORM 1503 03.82 (P04C01)